# EUROPEAN PATENT SPECIFICATION

(11) **EP 2 854 773 B1**
(45) Date of publication and mention of the grant of the patent: **06.05.2020**
(21) Application number: 13797878.9
(22) Date of filing: 28.05.2013
(51) Int. Cl.: A61K 9/16, A61J 3/10, A61K 31/522, A61P 31/20, A61K 9/20

(54) **PHARMACEUTICAL COMPOSITION OF ENTECAVIR AND PROCESS OF MANUFACTURING**
PHARMAZEUTISCHE ZUSAMMENSETZUNG VON ENTECAVIR UND VERFAHREN ZUR HERSTELLUNG
COMPOSITION PHARMACEUTIQUE D'ENTECAVIR, ET PROCÉDÉ DE FABRICATION

(30) Priority: 31.05.2012 CA 2779052
(43) Date of publication of application: 08.04.2015
(73) Proprietor: Pharmascience Inc., Montréal, Québec H4P 2T4 (CA)
(72) Inventor: PHILIP, Mathew, St. Laurent, Québec H4R 2A9 (CA); TALWAR, Naresh, Kirkland, Québec H9H 5L1 (CA)
(74) Representative: HGF Limited
(86) International application number: PCT/CA2013/000517
(87) International publication number: WO 2013/177672

(56) References cited:
- EP-A1- 1 145 711
- EP-A1- 2 508 172
- EP-A1- 2 508 172
- WO-A1-2009/106954
- WO-A1-2011/076412
- WO-A1-2012/068535
- WO-A1-2013/114389
- WO-A1-2013/114389
- WO-A2-2009/106960
- WO-A2-2013/072937
- WO-A2-2013/072937
- CA-A1- 2 401 569
- J. Fung ET AL: "Nucleoside/nucleotide analogues in the treatment of chronic hepatitis B", JOURNAL OF ANTIMICROBIAL CHEMOTHERAPY., vol. 66, no. 12, 29 September 2011 (2011-09-29), pages 2715-2725, XP055370530, GB ISSN: 0305-7453, DOI: 10.1093/jac/dkr388

## Description

### FIELD OF THE INVENTION

The present invention relates to an oral pharmaceutical composition for the treatment of hepatitis B virus infection. More specifically, the present invention is directed to adhesive-free pharmaceutical compositions and pharmaceutical compositions comprising granules that are adhesive-free and comprise a guanine-based antiviral active pharmaceutical ingredient and manufacturing process of said pharmaceutical composition.

### BACKGROUND OF THE INVENTION

Entecavir is an oral antiviral drug used in the treatment of hepatitis B infection. Entecavir is a guanosine nucleoside analogue with selective activity against hepatitis B virus (HBV), which inhibits reverse transcription, DNA replication and transcription in the viral replication process.

Entecavir also helps to prevent the hepatitis B virus from multiplying and infecting new liver cells, is also indicated for the treatment of chronic hepatitis B in adults with HIV/AIDS infection.

The chemical name for entecavir is 2-amino-I,9-dihydro-9-[(1*S*,3*R*,4*S*)-4-hydroxy-3 - (hydroxymethyl)-2-methylenecyclopentyl]-6*H*-purin-6-one, monohydrate. Its molecular formula is C₁₂H₁₅N₅O₃•H₂O, which corresponds to a molecular weight of 295.3.

Entecavir has the following structural formula:

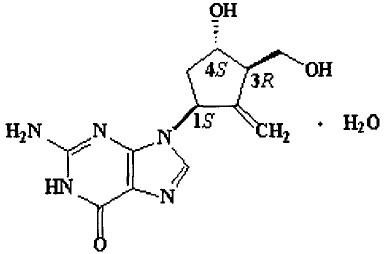

Entecavir is a slightly water soluble drug (2.4 mg/mL) and the pH of the saturated solution in water is 7.9 at 25° ± 0.5° C. This leads to great difficulty in formulating immediate release dosage forms containing low dose content of entacavir. This, in turn, it makes it difficult to develop a robust formulation and a process for manufacturing same.

Such low solubility can often result in poor dissolution behaviour, which can often result in low bioavailability, particularly given limited transit times through the gastrointestinal tract.

BARACLUDE® is a film-coated tablet containing entecavir. According to the World Standard Drug Database, the commercially available formulation of the BARACLUDE® film-coated tablets contain the following: entecavir as the active pharmaceutical ingredient (API) (0.5 mg and 1 mg) and the following inactive ingredients: lactose monohydrate, microcrystalline cellulose, crospovidone, povidone, and magnesium stearate. The tablet coating contains titanium dioxide, hypromellose, polyethylene glycol 400, polysorbate 80, and iron oxide red.

Preparations of entecavir are disclosed in the following references: Canadian Patent No. 2,053,339 (corresponds to U.S. Patent No. 5,206,244; and EP 0481754), International Publication No. WO 98/09964, Canadian Patent Application No. 2,569,484 (corresponds to International Publication No. WO2005/118585) and Canadian Patent Application No. 2,508,811 (corresponds to International Publication No. WO 2004/52310).

Various techniques to make entecavir-containing dosage forms are described in the following prior art: Canadian Patent No. 2,401,569 (corresponds to International Publication No. WO 2001/064221), U.S. Patent No. 6,627,224 (corresponds to EP1267880), Canadian Patent Application No. 2,462,886, Indian Patent Application No. IN2009MUM00913, and International Publication No. WO 2011/128623.

There is considerable interest among pharmaceutical scientists involved in this area of research to either modify the existing products or develop new materials with properties that satisfy as many requirements as possible. For example, see the article entitled, "Stability of low concentration of guanine based antivirals in sucrose or maltitol solutions" (International Journal of Pharmaceutics, 342 /2007, p. 87- 94).

Canadian Patent No. 2,053,339 (corresponds to U.S. Patent No. 5,206,244; and EP 0481754) discloses entecavir and its use in treating hepatitis B. This patent discloses that an effective antiviral dose for oral administration will be in the range of about 1.0 to 50 mg/kg of body weight and that the desired dose may be administered several times daily at appropriate intervals.

Indian Patent Application No. IN2009MUM00913 discloses pharmaceutical compositions of entecavir for oral treatment of hepatitis B virus infection and/or co-infections and also relates to processes of preparation of such compositions, particularly direct compression is disclosed.

International Publication No. WO 2011/128623 discloses a composition comprising at least one water-insoluble antiviral drug and at least one water-soluble carrier material, wherein the water-insoluble antiviral drug is dispersed through the water-soluble carrier material in nano-disperse form.

Canadian Patent Application No. 2,462,886 (corresponds to International Publication No. WO2003/030868) and Canadian Patent No. 2,311,734 disclose a flash-melt pharmaceutical dosage form comprising entecavir that rapidly disperses in the mouth, wherein a combination of superdisintegrants is utilized to enhance bioequivalence and stability. It discloses also that the flash-melt pharmaceutical dosage forms may be prepared by dry granulation of the excipients with the medicament and suitable conventional ingredients, such as flavouring and sweetening agents, without the use of any solvent, to form stable granules that can be readily compressed into dosage forms on conventional equipment without the need for special handling techniques.

Canadian Patent No. 2,401,569 (corresponds to International Publication No. WO 2001/064221 and EP 1267880) discloses a low dose entecavir formulation and uses thereof. The preferred pharmaceutical compositions contain from about 0.01 mg to about 10 mg of entecavir adhered to a pharmaceutically acceptable carrier substrate through the use of an adhesive substance, such as a polymeric material possessing a high degree of tackiness. Suitable adhesive materials include povidone, methylcellulose, hydroxymethylcellulose, hydroxypropylmethylcellulose, hydroxypropylcellulose, hydroxyethylcellulose, gelatin, guar gum, and xanthan gum and mixtures thereof with povidone being preferred. The compositions are prepared by first carefully depositing the entecavir on the surface of the carrier substrate particles. This step is accomplished by forming a solution of the entecavir in a solvent along with an adhesive substance at temperatures ranging from about 25°C to about 80°C and applying the solution as a spray or a stream while the carrier substrate particles are in motion. The conditions are controlled to minimize particle agglomeration. Subsequently, the solvent is removed from the carrier surface leaving the entecavir particles adhered to the surface of the carrier substrate. This prevents the separation of the entecavir from the substrate and minimizes the loss of entecavir during subsequent processing. The compositions taught are formulated for oral administration in the form of tablets or capsules, which further contain pharmaceutically acceptable excipients including bulking agents, lubricants, disintegrants, binding agents, etc. as commonly employed in such compositions. Such compositions cannot be prepared with good content uniformity by simply mixing the active substance and the excipients. The traditional methods of granulation are also not suitable for products containing active ingredients at such low doses.

Canadian Patent No. 2,401,569 discloses the manufacture of low dose entecavir formulations utilizing an adhesive agent to prevent the separation of entecavir from the substrate. The solubility of entecavir in purified water is 2.4 mg/ml which is low and hence would require large amounts of solvent to be incorporated to the carrier substrate. Adding large amounts of solvent along with adhesive agent onto a carrier substrate by traditional methods of granulation like high shear granulation could lead to processing issues (over-granulation, loss of porosity of granules, less compaction, low dissolution, etc). Adding an adhesive agent to the solvent to make the API adhere to the carrier substrate and have good content uniformity would increase the complexity of manufacturing due to more binding of the solution to the carrier substrate coupled with the shear of mixing. Incorporating high amounts of solvent could be achieved by spray granulation process where the carrier particles are fluidized and the solvent is sprayed at a constant rate on to the carrier substrate. There is also mention that the solubility of entecavir could be increased by adding pH adjusted water (by addition of acid or base in the purified water) which is not a common practice and could impart degradation to the product, since the drug is dissolved in the solvent. European Patent EP 2508172 discloses a tablet comprising entecavir prepared by wet-granulation process for the treatment of hepatitis B virus infections. The composition (Example 1) comprises Intragranular portion: Entecavir Monohydrate 0.4% Lactose Monohydrate 57.9% Avicel PH 101 15,8%, Crospovidone XL 10 6.8%, Extra-granular portion : Avicel PH 102 13.6%, Crospovidone XL10 1%, Mg Stearate 1.5%, and as coating Opadry YS-1-7003.

There are numerous other examples of specific formulations that utilize one or more of the techniques and methods discussed above. However, each one of those have some limitations related to the processing, either it is difficult or expensive to produce dosage forms by such techniques and resulting dosage forms are friable or are sensitive to environmental factors.

Therefore, there exists a need for a formulation providing entecavir in a low dose. The present invention provides an adhesive-free pharmaceutical composition containing entecavir by wet granulation and pharmaceutical compositions comprising granules that are adhesive free and comprise entecavir. The present invention therefore, in turn mitigates or eliminates these limitations during formulation and manufacturing.

### SUMMARY OF THE INVENTION

The present invention provides a pharmaceutical composition comprising:
a) granules comprising:
   i) entecavir or a pharmaceutically acceptable salt thereof;
   ii) at least one intra-granular pharmaceutically acceptable excipient, wherein the granules do not comprise povidone, methylcellulose, hydroxymethylcellulose, hydroxypropylmethylcellulose, hydroxypropylcellulose, hydroxyethylcellulose, gelatin, guar gum, and xanthan gum and mixtures thereof;
b) at least one extra-granular pharmaceutical excipient wherein an extra-granular pharmaceutical excipient is a lubricant selected from the group consisting of: magnesium stearate, calcium stearate, zinc stearate, sodium stearate, stearic acid, aluminum stearate, glyceryl behenate, hydrogenated vegetable oil and combinations thereof; c) a moisture barrier coating;
wherein said composition is suitable for the treatment of hepatitis B virus infection.

at least one intra-granular pharmaceutically acceptable excipient; at least one extra-granular pharmaceutical excipient, and optionally a moisture barrier coating, wherein said composition is used for the treatment of hepatitis B virus infection.

It was established that, in certain cases, entecavir tablets coated with moisture barrier coatings (for example, PVA-based Opadry®, Opadry® AMB, Opadry® 200, etc.) provided some additional stability compared to normal hydroxypropylmethylcellulose (HPMC) based coatings.

According to another aspect of the present there is provided a wet granulation process for manufacturing an adhesive-free pharmaceutical composition of entecavir; such process is potentially simpler and less expensive than prior art processes.

The use of adhesive-free pharmaceutical composition results in a less expensive and simpler entecavir formulation having good physical stability.

According to a further aspect of the present invention there is provided a pharmaceutical composition for oral administration comprising an adhesive-free granule which comprises at least one slightly soluble guanine-based antiviral active pharmaceutical ingredient and at least one intra-granular pharmaceutically acceptable excipient; at least one extra-granular pharmaceutical excipient, and optionally a moisture barrier coating, said adhesive-free pharmaceutical composition is used for the treatment of hepatitis B virus infection.

Preferably, the guanine-based antiviral active pharmaceutical ingredient of said pharmaceutical composition is entecavir or a pharmaceutically acceptable salt or solvate thereof.

The amount of entecavir in said composition ranges from about 0.1 mg to about 5.0 mg. In preferred embodiments of the present invention, the amount entecavir present is about 0.1 mg, about 0.5 mg and about 1.0 mg.

Another aspect of the present invention provides a pharmaceutical composition comprising entecavir or a pharmaceutically acceptable salt or solvate thereof, along with at least one pharmaceutically acceptable excipient selected from the group consisting of: fillers, diluents, lubricants, disintegrants, coating polymers and combinations thereof.

Preferably, the filler is selected from the group consisting of: cellulose, dibasic calcium phosphate, calcium carbonate, microcrystalline cellulose, sucrose, lactose, glucose, mannitol, sorbitol, maltol, pregelatinized starch, corn starch, potato starch and combinations thereof.

More preferably, the filler is lactose monohydrate and is present in an amount ranging from about 30% w/w to about 70% w/w of the total composition.

Also preferably, the filler is microcrystalline cellulose and is present in an amount ranging from about 30% w/w to about 70% w/w of the total composition.

Preferably, the disintegrant is selected from the group consisting of: crospovidone, sodium starch glycolate, sodium pregelatinized starch, modified corn starch and combinations thereof. More preferably, the disintegrant is crospovidone and is present in an amount ranging from about 2.0% w/w to about 10% w/w of the total composition.

Preferably, the lubricant is selected from the group consisting of: magnesium stearate, calcium stearate, zinc stearate, sodium stearate, stearic acid, aluminum stearate, glyceryl behenate, hydrogenated vegetable oil and combinations thereof.

More preferably, the lubricant is magnesium stearate and is present in an amount ranging from about 0.1% w/w to about 2% w/w of the total composition.

Further preferably, the moisture barrier coating is selected from the group consisting of: PVA-based Opadry®, Opadry®AMB, Opadry®200, and mixtures thereof.

More preferably, the present invention is directed to an adhesive-free pharmaceutical composition for oral administration comprising: entecavir, lactose, microcrystalline cellulose, crospovidone, magnesium stearate, and optionally moisture barrier coating, wherein said composition is in the form of a tablet or a capsule.

Another aspect of the present invention provides for a method of manufacturing an adhesive-free pharmaceutical composition comprising following steps:
(1) preparation of a granulation solution;
(2) granulation;
(3) drying;
(4) extra -granular mixing;
(5) lubrication, and
(6) compression.

Preferably, the method of manufacturing an adhesive-free pharmaceutical composition for oral administration comprising: adhesive-free granules comprising therapeutically effective amount of entecavir and at least one intra-granular pharmaceutically acceptable excipient; at least one extra-granular pharmaceutical excipient, and optionally a moisture barrier coating, wherein said process comprising following steps:
(1) dissolving entecavir in a hydro alcoholic solution containing dehydrated alcohol and purified water;
(2) preparing a granulation solution;
(3) adding lactose monohydrate, microcrystalline cellulose and crospovidone XL to high shear granulator and mixing;
(4) adding the granulation solution of step (2) to the mixing blend of step (3);
(5) rinsing container with dehydrated alcohol and purified water and adding this solution to the high shear bowl under mixing;
(6) drying the wet granules obtained from step (5);
(7) screening the dried granules of step (6) to obtain uniform lump free granules;
(8) adding the screened granules of step (7) to a bin blender;
(9) adding microcrystalline cellulose and crospovidone to the blend of step (8) and mixing;
(10) adding magnesium stearate on the granules of step (7);
(11) adding granules of step (10) to the blend of step (9) and mixing;
(12) compressing the content of step (11), and
(13) optionally coating the content of step (12) with coating dispersion.

The present invention also relates to the use of an adhesive-free pharmaceutical composition for the treatment of patients having hepatitis B virus infection, such compositions comprising entecavir in an amount ranging from about 0.1 mg to about 5.0 mg.

These and other aspects, advantages and features of the present invention are provided in detailed description and examples as follows.

### DETAILED DESCRIPTION OF THE INVENTION

The terms "active ingredient" and "active agent" (as well as other terms a person skilled in the art would be well aware of) refers to an active pharmaceutical ingredient (API) which is the active chemical used in the manufacturing of drugs. The active agent can be a therapeutic, a prophylactic, or a diagnostic agent.

The term "therapeutically effective amount" intends to describe an amount of the active agent which stops or reduces the progress of the condition intended to be treated or which otherwise completely or partly cures or acts palliative on the condition.

The term "adhesive-free granules" is used to describe granules containing the guanine-based antiviral active pharmaceutical ingredient with at least one intra-granular pharmaceutically acceptable excipient that are free of adhesive substances or materials. Adhesive substances include polymeric material possessing a high degree of tackiness. Suitable adhesive materials include povidone, methylcellulose, hydroxymethylcellulose, hydroxypropylmethylcellulose, hydroxypropylcellulose, hydroxyethylcellulose, gelatin, guar gum, and xanthan gum and mixtures thereof.

According to the present invention the guanine-based antiviral active pharmaceutical ingredient is selected from the group consisting of antiviral agents. Preferably, the antiviral agent is lamivudine, pegylated interferon, adefovir, entecavir, telbivudine, tenofovir and combinations thereof. More preferably, the antiviral agent is entecavir.

In addition to the guanine-based antiviral active pharmaceutical ingredient, the pharmaceutical composition according to the present invention contains at least one pharmaceutically acceptable excipient added to the composition for various purposes. At least one pharmaceutically acceptable excipient may be present in the formulation of the present invention, but not limited to: diluents, fillers, binders, lubricants, disintegrants, glidants, and acidifying agents. As understood by a person skilled in the art, these excipients are standard and well known in the pharmaceutical art.

The filler according to the present invention is selected from the group consisting of: cellulose, microcrystalline cellulose, dibasic calcium phosphate, calcium carbonate, sucrose, lactose, glucose, mannitol, sorbitol, maltol, pregelatinized starch, corn starch, potato starch and combinations thereof. Preferably, the filler is selected from the group consisting of: lactose monohydrate and microcrystalline cellulose, alone or in combination.

The disintegrant according to the present invention is selected from the group consisting of: crospovidone, sodium starch glycolate, sodium pregelatinized starch, modified corn starch and combinations thereof. Preferably, the disintegrant is crospovidone.

The lubricant according to the present invention is selected from the group consisting of: magnesium stearate, calcium stearate, zinc stearate, sodium stearate, stearic acid, aluminum stearate, glyceryl behenate, hydrogenated vegetable oil and combinations thereof. Preferably, the lubricant is magnesium stearate.

According to the present invention the moisture barrier coating is selected from the group consisting of: PVA-based Opadry®, Opadry®AMB, Opadry®200, and mixtures thereof. Opadry® is a registered trade mark of Colorcon Inc.

Furthermore, the present invention discloses a stable, adhesive-free, pharmaceutical composition of entecavir, having a USP acceptance value for content uniformity of 85% to 115 % by weight and a relative standard deviation (RSD) of less than 6 %.

Stability data in ALU/ALU cold forming blister at 40°C and 75% RH for 1 month, shows an unknown degradation product at RRT 1.98, which does not increase for the tablets coated with moisture barrier coatings as compared to tablets coated with normal non barrier coating system. These adhesive-free pharmaceutical compositions exhibit good content uniformity and stability.

The following Examples illustrate the preferred embodiments. The Examples in no way limit the scope of the present invention.

### EXAMPLE 1

### AN ADHESIVE-FREE PHARMACEUTICAL COMPOSITION OF ENTECAVIR

### Step 1: Preparation of granulation solution

The required quantity of entecavir (2.65 g) was dissolved in a hydro alcoholic solution containing 50:50 v/v (540.0g) of dehydrated alcohol and purified water under stirring at room temperature. Stirring was continued until a clear solution was obtained. This solution was used as granulating solution.

### Step 2: Granulation

Lactose monohydrate, microcrystalline cellulose and crospovidone XL were added to high shear granulator in required quantities (see Table 1) and mixed for 5 minutes. The granulating solution of step 1 was added to the high shear bowl under mixing. Once the solution was added completely the container was rinsed with 50:50 v/v dehydrated alcohol and purified water. This solution was also added to the high shear bowl under mixing.

### Step 3: Drying

The wet granules of step (2) were dried in a fluid bed until a loss on drying (LOD) value of 2-3% was obtained. Then, dried granules of previous step were screened through a 1100µm screen to obtain uniform lump free granules.

### Step 4: Extra granular mixing

The screened granules of step (3) were added to a bin blender. The required quantity of microcrystalline cellulose and crospovidone XL was adjusted based on the yield of granules of previous step. These ingredients were screened manually through a 425µm screen and were added to blender and blended for 10 minutes.

### Step 5: Lubrication

The required quantity of magnesium stearate (see Table 1) was adjusted based on the yield of granules and further screened manually through a 425µm screen and added to blender of step (4) and was blended for 2 minutes.

### Step 6: Compression

The obtained blend was compressed on a compression machine at an average weight of 200mg to obtain 0.5mg per tablet of entecavir (see Table 1).

The formulation and manufacturing steps of Example 1 is set out in Table 1.

**Table 1: Formulation and Manufacturing steps.**

| **S.No.** | **Ingredient** | **Example 1** | | | |
|---|---|---|---|---|---|
| | | Function | mg/unit | % w/w | Qty in g for 5000 tablets |
| **Intra-granular blend** | | | | | |
| 1 | Lactose Monohydrate | Filler | 92.0 | 46.00 | 460.0 |
| 2 | Microcrystalline cellulose | Filler | 83.47 | 41.75 | 417.3 |
| 3 | Crospovidone XL | Disintegrant | 8.0 | 4.00 | 40.0 |

| **Granulation** | | | | | |
|---|---|---|---|---|---|
| 4 | Dehydrated alcohol: Purified water (50:50 v/v) | Granulating solvent | | | 540.0 |
| 5 | Entecavir monohydrate equivalent to 0.5 mg of entecavir | API | 0.53 | 0.25 | 2.65 |

| **Extra -granular blend** | | | | | |
|---|---|---|---|---|---|
| 6 | Microcrystalline cellulose | Filler | 6.5 | 3.25 | 32.5 |
| 7 | Crospovidone XL | Disintegrant | 8.0 | 4.00 | 40.0 |

| **Lubrication** | | | | | |
|---|---|---|---|---|---|
| 8 | Magnesium stearate | Lubricant | 1.5 | 0.75 | 7.5 |
| | Total | | 200.0 | 100.00 | 1000.0 |

Tablets manufactured as per Example 1 further were coated with a moisture barrier coating system.

### ANALYSIS OF COMPRESSED TABLETS OF ENTECAVIR

Content uniformity of tablets was evaluated for 10 individual tablets. The results are summarized in Table 2.

**Table 2: Content uniformity results of entecavir tablets of Example 1.**

| **Example 1** | |
|---|---|
| | **% LC** |
| **1** | 100.0 |
| **2** | 100.5 |
| **3** | 100.4 |
| **4** | 99.1 |
| **5** | 99.8 |
| **6** | 99.6 |
| **7** | 99.4 |
| **8** | 100.1 |
| **9** | 98.9 |
| **10** | 99.9 |
| **Average** | 99.8 |
| **SD** | 0.5 |
| **RSD (%)** | 0.5% |
| **Min (%)** | 98.9 |
| **Max (%)** | 100.5 |
| **Acceptance value (L1)** | 1.2% (conforms) |

### EXAMPLE 2

### STABILITY OF AN ADHESIVE-FREE PHARMACEUTICAL COMPOSITION OF ENTECAVIR.

Tablets manufactured as per Example 1 were further coated with two different coating systems one with non-moisture barrier coating system and other with a moisture barrier coating system to evaluate stability of packaged finished product. A comparative stability data is summarized in Table 3.

Tablets coated with non-moisture barrier coating system (HPMC based Opadry®) were designated as Formulation A.

Tablets coated with moisture barrier coating system (PVA based Opadry®) were designated as Formulation B.

Tablets coated with moisture barrier coating system (Opadry® AMB) were designated as Formulation C.

Tablets coated with moisture barrier coating system (Opadry®200) were designated as Formulation D.

**Table 3. COMPARATIVE STUDY ON STABILITY**

| | Pack: Tablets packed in ALU/ALU cold forming blister | | | | | | | | |
|---|---|---|---|---|---|---|---|---|---|
| | Stability condition: 40°C/75% RH for 1 Month | | | | | | | | |
| | | non-moisture barrier coating system | | moisture barrier coating system | | | | | |
| Sr. No | Degradation product | Formulation A | | Formulation B | | Formulation C | | Formulation D | |
| | Known degradation product | | | | | | | | |
| | Comp 1 | Not Detected | | Not Detected | | Not Detected | | Not Detected | |

| | Unknown degradation product | | | | | | | | |
|---|---|---|---|---|---|---|---|---|---|
| | | RRT | % | RRT | % | RRT | % | RRT | % |
| | | 1.98 | 0.39 | 1.99 | 0.04 | 1.94 | 0.04 | 2.00 | 0.04 |

| | Total degradation products | | | | | | | | |
|---|---|---|---|---|---|---|---|---|---|
| | | 0.56 | | 0.09 | | 0.17 | | 0.09 | |

| | | | | | | | | | |
|---|---|---|---|---|---|---|---|---|---|
| **Note:** All four tested formulation degradation products (known and unknown) were below the reporting thresholds during initial analysis. | | | | | | | | | |

### Compound 1: 2-Amino- 6- hydroxypurine.

### RRT: relative retention time

**Comments:** The stability data in ALU/ALU cold forming blister at 40°C/75% RH for 1 month indicates that an unknown degradation product at RRT 1.98 remained relatively constant for the tablets coated with moisture barrier coatings in comparison to tablets coated with normal non-barrier coating system.

### EXAMPLE 3

### ADHESIVE-FREE PHARMACEUTICAL COMPOSITION OF ENTECAVIR-COMPARATIVE STUDY ON STABILITY

Tablets manufactured as per Example 1 were further coated with two different coating systems one with non-moisture barrier coating system and other with moisture barrier coating system to evaluate stability of packaged finished product. A comparative stability data is summarized in Table 4.

**Table 4. COMPARATIVE STUDY ON STABILITY**

| | Pack: 30 Tablets packed in 60cc HDPE bottles | | | | | | | | |
|---|---|---|---|---|---|---|---|---|---|
| | Stability condition: 40°C/75% RH for 1 Month | | | | | | | | |
| | | Non-moisture barrier coating system | | Moisture barrier coating system | | | | | |
| Sr. No | Degradation product | Formulation A | | Formulation B | | Formulation C | | Formulation D | |
| | Known degradation product | | | | | | | | |
| | Comp 1 | Not detected | | Not detected | | Not detected | | Not detected | |

| | Unknown degradation product | | | | | | | | |
|---|---|---|---|---|---|---|---|---|---|
| | | **RRT** | **%** | **RRT** | **%** | **RRT** | **%** | **RRT** | **%** |
| | | 0.49 | 0.08 | 1.96 | 0.05 | 1.93 | 0.03 | 1.98 | 0.07 |
| | | 1.86 | 0.49 | | | | | | |
| | | 2.41 | 0.08 | | | | | | |

| | Total degradation products | | | | | | | | |
|---|---|---|---|---|---|---|---|---|---|
| | | 0.85 | | 0.17 | | 0.19 | | 0.18 | |

| | | | | | | | | | |
|---|---|---|---|---|---|---|---|---|---|
| **Note:** For all four tested formulation degradation products (known and unknown) were below the reporting thresholds during initial analysis. | | | | | | | | | |

### Compound 1: 2-Amino- 6- hydroxypurine.

### RRT: relative retention time.

Formulation A - tablets coated with non-moisture barrier coating system (HPMC based Opadry®);
Formulation B -tablets coated with moisture barrier coating system (PVA based Opadry®):
Formulation C- tablets coated with moisture barrier coating system (Opadry® AMB);
Formulation D- tablets coated with moisture barrier coating system (Opadry®200).

**Comments:** The stability data in HDPE bottle at 40°C/75% RH for 1 month, indicates an unknown degradation product at RRT 1.98 which does not increase for the tablets coated with moisture barrier coatings in comparison to tablets coated with normal non-barrier coating system.

## Claims

1. A pharmaceutical composition comprising:
a) granules comprising:
i) entecavir or a pharmaceutically acceptable salt thereof;
ii) at least one intra-granular pharmaceutically acceptable excipient, wherein the granules do not comprise povidone, methylcellulose, hydroxymethylcellulose, hydroxypropylmethylcellulose, hydroxypropylcellulose, hydroxyethylcellulose, gelatin, guar gum, and xanthan gum and mixtures thereof;
b) at least one extra-granular pharmaceutical excipient wherein an extra-granular pharmaceutical excipient is a lubricant selected from the group consisting of: magnesium stearate, calcium stearate, zinc stearate, sodium stearate, stearic acid, aluminum stearate, glyceryl behenate, hydrogenated vegetable oil and combinations thereof;
c) a moisture barrier coating;
wherein said composition is suitable for the treatment of hepatitis B virus infection.

2. The pharmaceutical composition of claim 1, wherein entecavir is present in an amount ranging from about 0.1 mg to about 5.0 mg.

3. The pharmaceutical composition according to claim 2, wherein the composition comprises about 0.1 mg of entecavir or about 0.5 mg of entecavir or about 1.0 mg of entecavir.

4. The pharmaceutical composition according to any preceding claim, wherein the pharmaceutically acceptable excipients are selected from the group consisting of: fillers, diluents, lubricants, disintegrants, coating polymers and combinations thereof.

5. The pharmaceutical composition according to claim 4, wherein the filler is selected from the group consisting of: microcrystalline cellulose, cellulose, dibasic calcium phosphate, calcium carbonate, sucrose, lactose, glucose, mannitol, sorbitol, maltol, pregelatinized starch, corn starch, potato starch and combinations thereof, optionally wherein the filler is lactose monohydrate, or microcrystalline cellulose;
preferably wherein lactose is present in an amount ranging from about 30% w/w to about 70% w/w of the total composition or microcrystalline cellulose is present in an amount ranging from about 30% w/w to about 70% w/w of the total composition.

6. The pharmaceutical composition according to claim 4, wherein the disintegrant is selected from the group consisting of: crospovidone, sodium starch glycolate, sodium pregelatinized starch, modified corn starch and combinations thereof, optionally wherein the disintegrant is crospovidone; preferably wherein crospovidone is present in an amount ranging from about 2.0% w/w to about 10% w/w of the total composition.

7. The pharmaceutical composition according to any one of claims 1 to 3, wherein the lubricant is magnesium stearate.

8. The pharmaceutical composition according to any preceding claim, wherein the intra-granular pharmaceutical excipients comprise filler, optionally comprising microcrystalline cellulose and lactose, and disintegrant optionally comprising crospovidone.

9. The pharmaceutical composition according to any one of claims 1 to 3 and 8, wherein the extra-granular pharmaceutical excipients comprise filler, optionally comprising microcrystalline cellulose, and disintegrant, optionally comprising crospovidone.

10. The pharmaceutical composition according to claim 1, wherein the lubricant is magnesium stearate and is present in an amount ranging from about 0.1 % w/w to about 2% w/w of the total composition.

11. The pharmaceutical composition according to any preceding claim, wherein said pharmaceutical composition is a tablet or a capsule.

12. The pharmaceutical composition according to any preceding claim, wherein said composition is manufactured by wet granulation method.

13. A pharmaceutical composition according to claim 1 comprising entecavir or a pharmaceutically acceptable salt thereof, present in an amount ranging from 0.1 mg to 5.0 mg, for use in the treatment of hepatitis B virus infection.

14. A method of manufacturing the pharmaceutical composition of claim 1, wherein said method comprises the following steps:
(1) dissolving entecavir in a hydro alcoholic solution containing dehydrated alcohol and purified water;
(2) preparing a granulation solution;
(3) adding filler and disintegrant to high shear granulator and mixing;
(4) adding the granulation solution of step (2) to the mixing blend of step (3);
(5) rinsing container with dehydrated alcohol and purified water and adding this solution to the high shear bowl under mixing;
(6) drying the wet granules obtained from step (5);
(7) screening the dried granules of step (6) to obtain uniform lump free granules;
(8) adding granules of step (7) to a bin blender;
(9) adding filler and disintegrant to the blend of step (8) and mixing;
(10) adding lubricant to the granules of step (7);
(11) adding granules of step (10) to the blend of step (9) and mixing;
(12) compressing the content of step (11); and
(13) coating the content of step (12) with coating dispersion.

15. The method of manufacturing a pharmaceutical composition according to claim 14, wherein: the filler included in step (3) comprises lactose and microcrystalline cellulose; and/or the filler included in step (9) comprises microcrystalline cellulose; and/or the disintegrant is crospovidone; and/or the lubricant is magnesium stearate.

## Patentansprüche

1. Pharmazeutische Zusammensetzung, umfassend:
a) Granulat umfassend:
i) Entecavir oder ein pharmazeutisch annehmbares Salz davon;
ii) mindestens einen intra-granulären pharmazeutisch annehmbaren Hilfsstoff, wobei das Granulat kein Povidon, Methylcellulose, Hydroxymethylcellulose, Hydroxypropylmethylcellulose, Hydroxypropylcellulose, Hydroxyethylcellulose, Gelatine, Guarkernmehl und Xanthangummi und keine Gemische davon umfasst;
b) mindestens einen extragranularen pharmazeutischen Hilfsstoff, wobei ein extragranularer pharmazeutischer Hilfsstoff ein Schmiermittel ist, ausgewählt aus der Gruppe bestehend aus: Magnesiumstearat, Calciumstearat, Zinkstearat, Natriumstearat, Stearinsäure, Aluminiumstearat, Glycerylbehenat, hydriertem Pflanzenöl und Kombinationen davon;
c) eine Feuchtigkeitssperrbeschichtung;
wobei die Zusammensetzung für die Behandlung einer Hepatitis-B-Virusinfektion geeignet ist.

2. Pharmazeutische Zusammensetzung nach Anspruch 1, wobei Entecavir in einer Menge im Bereich von etwa 0,1 mg bis etwa 5,0 mg vorhanden ist.

3. Pharmazeutische Zusammensetzung nach Anspruch 2, wobei die Zusammensetzung etwa 0,1 mg Entecavir oder etwa 0,5 mg Entecavir oder etwa 1,0 mg Entecavir umfasst.

4. Pharmazeutische Zusammensetzung nach einem der vorherigen Ansprüche, wobei die pharmazeutisch annehmbaren Hilfsstoffe ausgewählt sind aus der Gruppe bestehend aus: Füllstoffen, Verdünnungsmitteln, Schmiermitteln, Sprengmitteln, Beschichtungspolymeren und Kombinationen davon.

5. Pharmazeutische Zusammensetzung nach Anspruch 4, wobei der Füllstoff ausgewählt ist aus der Gruppe bestehend aus: mikrokristalliner Cellulose, Cellulose, dibasischem Calciumphosphat, Calciumcarbonat, Saccharose, Lactose, Glucose, Mannit, Sorbit, Maltol, Quellstärke, Maisstärke, Kartoffelstärke und Kombinationen davon, optional wobei der Füllstoff Lactosemonohydrat oder mikrokristalline Cellulose ist;
vorzugsweise wobei Lactose in einer Menge im Bereich von etwa 30 % Gew./Gew. bis etwa 70 % Gew./Gew. der Gesamtzusammensetzung vorhanden ist oder mikrokristalline Cellulose in einer Menge im Bereich von etwa 30 % Gew./Gew. bis etwa 70 % Gew./Gew. der Gesamtzusammensetzung vorhanden ist.

6. Pharmazeutische Zusammensetzung nach Anspruch 4, wobei das Sprengmittel ausgewählt ist aus der Gruppe bestehend aus: Crospovidon, Natriumstärkeglykolat, vorgelatinierter Natriumstärke, modifizierter Maisstärke und Kombinationen davon, optional wobei das Sprengmittel Crospovidon ist; vorzugsweise wobei Crospovidon in einer Menge im Bereich von etwa 2,0 % Gew./Gew. bis etwa 10 % Gew./Gew. der Gesamtzusammensetzung vorhanden ist.

7. Pharmazeutische Zusammensetzung nach einem der Ansprüche 1 bis 3, wobei das Schmiermittel Magnesiumstearat ist.

8. Pharmazeutische Zusammensetzung nach einem der vorherigen Ansprüche, wobei die intragranularen pharmazeutischen Hilfsstoffe Füllstoff, optional umfassend mikrokristalline Cellulose und Lactose, und optional Sprengmittel, umfassend Crospovidon, umfassen.

9. Pharmazeutische Zusammensetzung nach einem der Ansprüche 1 bis 3 und 8, wobei die extra-granularen pharmazeutischen Hilfsstoffe Füllstoff, optional umfassend mikrokristalline Cellulose, und Sprengmittel, optional umfassend Crospovidon, umfassen.

10. Pharmazeutische Zusammensetzung nach Anspruch 1, wobei das Schmiermittel Magnesiumstearat ist und in einer Menge im Bereich von etwa 0,1 % Gew./Gew. bis etwa 2 % Gew./Gew. der Gesamtzusammensetzung vorhanden ist.

11. Pharmazeutische Zusammensetzung nach einem vorherigen Anspruch, wobei die pharmazeutische Zusammensetzung eine Tablette oder eine Kapsel ist.

12. Pharmazeutische Zusammensetzung nach einem der vorhergehenden Ansprüche, wobei die Zusammensetzung durch ein Nassgranulierungsverfahren hergestellt ist.

13. Pharmazeutische Zusammensetzung nach Anspruch 1, umfassend Entecavir oder ein pharmazeutisch annehmbares Salz davon, das in einer Menge im Bereich von 0,1 mg bis 5,0 mg vorhanden ist, zur Verwendung bei der Behandlung einer Hepatitis-B-Virusinfektion.

14. Verfahren zum Herstellen der pharmazeutischen Zusammensetzung nach Anspruch 1, wobei das Verfahren die folgenden Schritte umfasst:
(1) Auflösen von Entecavir in einer hydroalkoholischen Lösung, die dehydrierten Alkohol und aufgereinigtes Wasser enthält;
(2) Herstellen einer Granulierungslösung;
(3) Hinzufügen von Füllstoff und Sprengmittel zu dem High-Shear-Granulierer und Mischen;
(4) Hinzufügen der Granulierungslösung von Schritt (2) zu dem Mischgemisch von Schritt (3);
(5) Spülen des Behälters mit dehydriertem Alkohol und aufgereinigtem Wasser und Hinzufügen dieser Lösung in die High-Shear-Schüssel unter Mischen;
(6) Trocknen des aus Schritt (5) erlangten nassen Granulats;
(7) Sieben des getrockneten Granulats von Schritt (6), um ein einheitliches klumpenfreies Granulat zu erlangen;
(8) Hinzufügen von Granulat von Schritt (7) zu einem Behälter-Mischer;
(9) Hinzufügen von Füllstoff und Sprengmittel zu dem Gemisch von Schritt (8) und Mischen;
(10) Hinzufügen von Schmiermittel zu dem Granulat von Schritt (7);
(11) Hinzufügen von Granulat von Schritt (10) zu dem Gemisch von Schritt (9) und Mischen;
(12) Komprimieren des Inhalts von Schritt (11); und
(13) Beschichten des Inhalts von Schritt (12) mit Beschichtungsdispersion.

15. Verfahren zum Herstellen einer pharmazeutischen Zusammensetzung nach Anspruch 14, wobei: der in Schritt (3) beinhaltete Füllstoff Lactose und mikrokristalline Cellulose umfasst; und/oder der in Schritt (9) enthaltene Füllstoff mikrokristalline Cellulose umfasst; und/oder das Sprengmittel Crospovidon ist; und/oder das Schmiermittel Magnesiumstearat ist.

## Revendications

1. Composition pharmaceutique comprenant :
a) des granulés comprenant :
i) de l'entécavir ou un sel pharmaceutiquement acceptable de celui-ci ;
ii) au moins un excipient intra-granulaire pharmaceutiquement acceptable, dans lequel les granulés ne comprennent pas de povidone, de méthylcellulose, d'hydroxyméthylcellulose, d'hydroxypropylméthylcellulose, d'hydroxypropylcellulose, d'hydroxyéthylcellulose, de gélatine, de gomme de guar et de gomme de xanthane et leurs mélanges ;
b) au moins un excipient pharmaceutique extra-granulaire dans lequel un excipient pharmaceutique extra-granulaire est un lubrifiant choisi dans le groupe comprenant : le stéarate de magnésium, le stéarate de calcium, le stéarate de zinc, le stéarate de sodium, l'acide stéarique, le stéarate d'aluminium, le béhénate de glycéryle, l'huile végétale hydrogénée et leurs combinaisons ;
c) un revêtement formant barrière à l'humidité ;
dans laquelle ladite composition convient au traitement de l'infection par le virus de l'hépatite B.

2. Composition pharmaceutique selon la revendication 1, dans laquelle l'entécavir est présent en une quantité allant d'environ 0,1 mg à environ 5,0 mg.

3. Composition pharmaceutique selon la revendication 2, dans laquelle la composition comprend environ 0,1 mg d'entécavir ou environ 0,5 mg d'entécavir ou environ 1,0 mg d'entécavir.

4. Composition pharmaceutique selon l'une quelconque des revendications précédentes, dans laquelle les excipients pharmaceutiquement acceptables sont choisis dans le groupe constitué de : charges, diluants, lubrifiants, délitants, polymères d'enrobage et leurs combinaisons.

5. Composition pharmaceutique selon la revendication 4, dans laquelle la charge est choisie dans le groupe constitué de : cellulose microcristalline, cellulose, phosphate de calcium dibasique, carbonate de calcium, saccharose, lactose, glucose, mannitol, sorbitol, maltol, amidon prégélatinisé, amidon de maïs, amidon de pomme de terre et leurs combinaisons, facultativement dans laquelle la charge est le lactose monohydraté ou la cellulose microcristalline ;
de préférence dans laquelle le lactose est présent en une quantité allant d'environ 30 % en poids à environ 70 % en poids de la composition totale ou la cellulose microcristalline est présente en une quantité allant d'environ 30 % en poids à environ 70 % en poids de la composition totale.

6. Composition pharmaceutique selon la revendication 4, dans laquelle le délitant est choisi dans le groupe constitué de : crospovidone, glycolate d'amidon sodique, amidon prégélatinisé sodique, amidon de maïs modifié et leurs combinaisons, facultativement dans laquelle le délitant est la crospovidone ; de préférence dans laquelle la crospovidone est présente en une quantité allant d'environ 2,0 % en poids à environ 10 % en poids de la composition totale.

7. Composition pharmaceutique selon l'une quelconque des revendications 1 à 3, dans laquelle le lubrifiant est le stéarate de magnésium.

8. Composition pharmaceutique selon l'une quelconque des revendications précédentes, dans laquelle les excipients pharmaceutiques intra-granulaires comprennent une charge, comprenant facultativement de la cellulose microcristalline et du lactose, et un délitant comprenant facultativement de la crospovidone.

9. Composition pharmaceutique selon l'une quelconque des revendications 1 à 3 et 8, dans laquelle les excipients pharmaceutiques extra-granulaires comprennent une charge, comprenant facultativement de la cellulose microcristalline, et un délitant, comprenant facultativement de la crospovidone.

10. Composition pharmaceutique selon la revendication 1, dans laquelle le lubrifiant est le stéarate de magnésium et est présent en une quantité allant d'environ 0,1 % en poids à environ 2 % en poids de la composition totale.

11. Composition pharmaceutique selon l'une quelconque des revendications précédentes, dans laquelle ladite composition pharmaceutique est un comprimé ou une capsule.

12. Composition pharmaceutique selon l'une quelconque des revendications précédentes, dans laquelle ladite composition est fabriquée par un procédé de granulation humide.

13. Composition pharmaceutique selon la revendication 1 comprenant de l'entécavir ou un sel pharmaceutiquement acceptable de celui-ci, présent en une quantité allant de 0,1 mg à 5,0 mg, pour une utilisation dans le traitement d'une infection par le virus de l'hépatite B.

14. Procédé de fabrication de la composition pharmaceutique selon la revendication 1, dans lequel ledit procédé comprend les étapes suivantes :
(1) dissolution de l'entécavir dans une solution hydroalcoolique contenant de l'alcool déshydraté et de l'eau purifiée ;
(2) préparation d'une solution de granulation ;
(3) ajout d'une charge et d'un délitant au granulateur à cisaillement élevé et mélange ;
(4) ajout de la solution de granulation de l'étape (2) au mélange de mélange de l'étape (3) ;
(5) rinçage du récipient avec de l'alcool déshydraté et de l'eau purifiée et ajout de cette solution au bol à cisaillement élevé en mélangeant ;
(6) séchage des granulés humides obtenus à l'étape (5) ;
(7) tamisage des granulés séchés de l'étape (6) pour obtenir des granulés uniformes sans grumeaux ;
(8) ajout des granulés de l'étape (7) à un mélangeur à bacs ;
(9) ajout d'une charge et d'un délitant au mélange de l'étape (8) et mélange ;
(10) ajout de lubrifiant aux granulés de l'étape (7) ;
(11) ajout des granulés de l'étape (10) au mélange de l'étape (9) et mélange ;
(12) compression du contenu de l'étape (11) ; et
(13) revêtement du contenu de l'étape (12) d'une dispersion de revêtement.

15. Procédé de fabrication d'une composition pharmaceutique selon la revendication 14, dans lequel : la charge incluse dans l'étape (3) comprend du lactose et de la cellulose microcristalline ; et/ou la charge incluse dans l'étape (9) comprend de la cellulose microcristalline ; et/ou le délitant est la crospovidone ; et/ou le lubrifiant est le stéarate de magnésium.
